# EUROPEAN PATENT SPECIFICATION

(11) **EP 4 176 806 B1**
(45) Date of publication and mention of the grant of the patent: **02.12.2026**
(21) Application number: 21853302.4
(22) Date of filing: 21.07.2021
(51) Int. Cl.: A61B 5/055, A61B 5/026, A61B 5/00, G16H 50/30, G16H 30/40

(54) **SYSTEM FOR ANALYSING RISK OF RUPTURE OF CEREBRAL ANEURYSM**
SYSTEM ZUR ANALYSE DES RISIKOS EINES BRUCHES EINES ZEREBRALEN ANEURYSMAS
SYSTÈME D'ANALYSE DU RISQUE DE RUPTURE D'UN ANÉVRISME CÉRÉBRAL

(30) Priority: 05.08.2020 KR 20200097740
(43) Date of publication of application: 10.05.2023
(73) Proprietor: Jeong, Seul Ki, Seoul 06294 (KR)
(72) Inventor: LEE, Chan Hyuk, Jeonju-si Jeollabuk-do 54906 (KR); JEONG, Seul Ki, Seoul 06294 (KR)
(74) Representative: Walaski, Jan Filip
(86) International application number: PCT/KR2021/009398
(87) International publication number: WO 2022/030822

(56) References cited:
- CN-A- 111 415 321
- KR-A- 20140 014 605
- KR-A- 20160 031 775
- MORI FUTOSHI ET AL: "Computational Fluid Dynamics Analysis of Lateral Striate Arteries in Acute Ischemic Stroke Using 7T High-resolution Magnetic Resonance Angiography", JOURNAL OF STROKE AND CEREBROVASCULAR DISEASES, DEMOS PUBLICATIONS, NEW YORK, NY, US, vol. 28, no. 11, 24 August 2019 (2019-08-24), XP085883181, ISSN: 1052-3057, [retrieved on 20190824], DOI: 10.1016/J.JSTROKECEREBROVASDIS.2019.104339
- ZULEGER D I ET AL: "Combining magnetic resonance measurements with numerical simulations - Extracting blood flow physiology information relevant to the investigation of intracranial aneurysms in the circle of Willis", INTERNATIONAL JOURNAL OF HEAT AND FLUID FLOW, BUTTERWORTH SCIENTIFIC LTD, GUILDFORD, GB, vol. 31, no. 6, 1 December 2010 (2010-12-01), pages 1032 - 1039, XP027501862, ISSN: 0142-727X, [retrieved on 20101116], DOI: 10.1016/J.IJHEATFLUIDFLOW.2010.07.003
- LEE WOO-JIN ET AL: "Impact of Endothelial Shear Stress on the Bilateral Progression of Unilateral Moyamoya Disease", STROKE, vol. 51, no. 3, 1 March 2020 (2020-03-01), US, pages 775 - 783, XP093178938, ISSN: 0039-2499, DOI: 10.1161/STROKEAHA.119.028117
- LIEBESKIND DAVID S. ET AL: "Noninvasive Fractional Flow on MRA Predicts Stroke Risk of Intracranial Stenosis : MRA Fractional Flow Predicts Stroke", JOURNAL OF NEUROIMAGING, vol. 25, no. 1, 5 March 2014 (2014-03-05), US, pages 87 - 91, XP055894215, ISSN: 1051-2284, Retrieved from the Internet <URL:https://api.wiley.com/onlinelibrary/tdm/v1/articles/10.1111%2Fjon.12101> DOI: 10.1111/jon.12101
- LIEBESKIND DAVID S., KOSINSKI ANDRZEJ S., LYNN MICHAEL J., SCALZO FABIEN, FONG ALBERT K., FARIBORZ PARI, CHIMOWITZ MARC I., FELDMA: "Noninvasive Fractional Flow on MRA Predicts Stroke Risk of Intracranial Stenosis : MRA Fractional Flow Predicts Stroke", JOURNAL OF NEUROIMAGING, LITTLE, BROWN AND CO., BOSTON, US, vol. 25, no. 1, 1 January 2015 (2015-01-01), US , pages 87 - 91, XP055894215, ISSN: 1051-2284, DOI: 10.1111/jon.12101
- WANG YUZHAO, GAO DUO, LIU HUAIJUN: "Evaluation of Cerebral Blood Flow Dynamics in Transient Ischemic Attacks Patients with Fast Cine Phase Contrast Magnetic Resonance Angiography", COMPUTATIONAL AND MATHEMATICAL METHODS IN MEDICINE, TAYLOR & FRANCIS, vol. 2020, 13 April 2020 (2020-04-13), pages 1 - 6, XP055894220, ISSN: 1748-670X, DOI: 10.1155/2020/4097829
- IBRAHIM A.Y., AMIRABADI A., SHROFF M.M., DLAMINI N., DIRKS P., MUTHUSAMI P.: "Fractional Flow on TOF-MRA as a Measure of Stroke Risk in Children with Intracranial Arterial Stenosis", AMERICAN JOURNAL OF NEURORADIOLOGY, US, vol. 41, no. 3, 1 March 2020 (2020-03-01), US , pages 535 - 541, XP055894223, ISSN: 0195-6108, DOI: 10.3174/ajnr.A6441
- HAN KAP-SOO, LEE SANG HYUK, RYU HAN UK, PARK SE-HYOUNG, CHUNG GYUNG-HO, CHO YOUNG I., JEONG SEUL-KI: "Direct Assessment of Wall Shear Stress by Signal Intensity Gradient from Time-of-Flight Magnetic Resonance Angiography", BIOMED RESEARCH INTERNATIONAL, HINDAWI PUBLISHING CORPORATION, vol. 2017, 1 January 2017 (2017-01-01), pages 1 - 8, XP055894227, ISSN: 2314-6133, DOI: 10.1155/2017/7087086

## Description

### Technical Field

The present disclosure relates to magnetic resonance angiography (MRA) application technology, and more particularly, to a method and a system for calculating blood flow and an index related thereto by analyzing a vascular image in MRA, and analyzing a risk of cerebrovascular disease or stroke by using the same.

### Background Art

A cerebrovascular disease and a stroke (an ischemic stroke or hemorrhagic stroke) are diseases that are caused by pathological phenomena such as stenosis, occlusion, rupture, or degeneration. From a viewpoint of blood flow, such a disease may be expressed by rapid reduction in blood flow (cerebral infarction) or persistently low blood flow (a cerebrovascular disease, vascular dementia) and a blood leak to the outside of a blood vessel.

The brain should maintain blood flow of 50 ml/min/100g or more in order to do normal brain activity. If blood flow abruptly decreases to less than 20-30 ml/min/100g, ischemic infarct may occur. In addition, if a patient has poor cerebral blood flow, about 30-50 ml/min/100g, the patient may suffer from a degenerative brain disease due to a chronic lack of energy (oxygen and glucose). A small vessel disease, dementia, and other neurodegenerative diseases are also caused by reduction in blood flow. In the case of cerebral hemorrhage, a change in blood flow around a hematoma is known as an important prognostic factor, and it is known that a cerebral aneurysm causing subarachnoid hemorrhage incurs various changes in blood flow after rupture.

Measurement of cerebral blood flow, particularly, exact and repetitive measurement according to a clinical variety of a patient is essential for initial diagnosis and long-term prognosis. In addition, measurement of cerebral blood flow may be utilized as a predictor of occurrence of a disease, and its utilization rate is high. A representative method of measuring cerebral blood flow is a method of quantitatively measuring by using an ultrasound examination or a phase contrast magnetic resonance (MR) technique, and the local blood flow distribution of brain may be measured by single photon emission computed tomography (SPECT) or blood oxygen-level dependent (BOLD) MRI. The ultrasound examination may obtain respective blood flows of four blood vessels such as left and right common carotid arteries and left and right vertebral arteries by measuring blood flow velocities and diameters of the blood vessels, and may obtain the total cerebral blood flow by adding up the blood flows. The phase contrast MR may also obtain the total cerebral blood flow by adding up values that are obtained by performing MR imaging for four blood vessels.

The ultrasound examination is most widely utilized in the clinical setting, but is highly dependent on examiner' experience. Particularly, it is not easy to examine vertebral arteries with the ultrasound examination, and it is difficult to exactly identify vascular variations. The phase contrast MR has an advantage of exactly measuring a blood flow velocity, but may not exactly measure a structure or a diameter of a blood vessel. Most of all, the phase contrast MR has the disadvantage that an examination should be individually performed for each blood vessel, and long time is required to scan. Therefore, the phase contrast MR is underutilized. There is a need for a method for measuring blood flow of four or more blood vessels simultaneously with a single examination within a short time in a clinical setting.

Mori Futoshi et al: "Computational Fluid Dynamics Analysis of Lateral Striate Arteries in Acute Ischemic Stroke Using 7T High-resolution Magnetic Resonance Angiography", Journal of Stroke and Cerebrovascular Diseases, Demos Publications, New York, NY, US, vol. 28, no. 11, 24 August 2019, XP085883181, ISSN: 1052-3057, DOI: 10.1016/J.JSTROKECEREBROVASDIS.2019.104339 discloses using computational fluid dynamics analysis of lateral striate arteries in acute ischemic stroke using 7T high-resolution magnetic resonance angiography.

A system for analyzing a risk of a rupture of a cerebral aneurysm is known from CN 111 415 321.

### Disclosure

### Technical Problem

The present disclosure has been developed in order to address the above-discussed deficiencies of the prior art, and an object of the present disclosure is to provide a method for calculating a signal intensity gradient-blood flow (SIG-BF) of a blood vessel site and factors related thereto, such as a SIG-velocity (SIG-V), a SIG-total cerebral blood flow (SIG-tCBF), a SIG-parenchymal cerebral blood flow (SIG-pCBF), a SIG-collateral blood flow (SIG-colBF), ΔSIG ratio and a SIG-BF ratio, by using a SIG in an MRA image, and a method and a system for diagnosing, predicting, and monitoring a cerebrovascular disease and a stroke by using the same.

### Technical Solution

A system for analyzing a risk of cerebrovascular disease according to claim 1 is provided. Optional features are set out in the dependent claims.

### Advantageous Effects

According to embodiments of the present disclosure, qualitative/quantitative analysis on cerebral hemodynamics and blood flow is possible by extracting a SIG-BF of a blood vessel site and factors related thereto, such as a SIG-V, a SIG-tCBF, a SIG-pCBF, a SIG-colBF, a ΔSIG ratio, and a SIG-BF ratio, by using a SIG in an MRA image.

In addition, according to embodiments of the disclosure, accurate diagnosis and monitoring of cerebral infarction, cerebrovascular disease, cerebral hemorrhage, etc. may be achieved by using the above-described factors according to various characteristics of vascular diseases, the mid- to long-term prognosis may be determined and the optimal treatment or treatment method may be selected, and future risks may be predicted.

### Description of Drawings

FIG. 1 is a view to explain a SIG-tCBF measurement method utilizing MRA;
FIG. 2 is a view illustrating an example showing SIG-pCBF;
FIGS. 3 to 5 are views to explain a method for measuring an aneurysm;
FIG. 6 is a view illustrating vascular images of a 74-year-old asymptomatic woman and a 37-year-old subarachnoid hemorrhage man;
FIG. 7 is a view illustrating a result of performing time-of-flight magnetic resonance angiography (TOF MRA) for a 56-year-old woman patient having an asymptomatic aneurysm, and its SIG display showing distributions of SIG values;
FIG. 8 is a block diagram of a cerebrovascular disease risk analysis system according to an embodiment.

### Best Mode

Hereinafter, the present disclosure will be described in more detail with reference to the drawings.

### 1. SIG-BF/SIG-V

Information obtained through computed tomography (CT), MR, or invasive angiography is expressed in a maximum intensity projection (MIP) method, showing blood vessels in white color in a black background. This method places an emphasis on anatomical analysis of blood vessels (for example, stenosis). Like other angiography techniques, TOF-MRA also provides an MIP image in a clinical setting.

However, since a signal intensity gradient (SIG) may be induced from TOF-MRA, embodiments of the present disclosure propose a method of deriving characteristics of blood flow reaching a blood vessel through a SIG and imaging the characteristics.

Specifically, embodiments of the present disclosure propose a method of deriving blood flow by using blood flow characteristics, and measuring the risk of stroke and cerebrovascular disease qualitatively/quantitatively by using the blood flow, thereby enhancing accuracy of diagnosis and a degree of prediction of disease, and utilizing the blood flow **in** monitoring before and after treatment and **in** determining a treatment method.

A signal intensity gradient-blood flow (SIG-BF) proposed **in** embodiments of the present disclosure will be described. First, a shear stress (*τ*) is defined by Equation 1 presented below according to the Poiseuille equation:

[Equation 1] $τ = 4 Qη / \left({πr}^{3}\right)$
where *τ* is a shear stress, Q is a blood flow, *η* is a blood viscosity, and *r* is a radius of a blood vessel.

In Equation 1, if the shear stress (*τ*) is replaced with the SIG, a blood flow (Qartery) on a blood wall of a corresponding blood vessel may be converted into Equation 2 presented below by applying the SIG:

[Equation 2] $Qartery = \left({τπr}^{3}\right) / 4 η ≒ {SIGπr}^{3} \left(unit,SI⋅{cm}^{2}\right)$

In Equation 2, SIG refers to a SIG (a maximum, a minimum, an average, etc.) on a blood wall of a corresponding blood vessel, and a blood flow obtained through the SIG is referred to as a SIG-BF.

Meanwhile, if *Qartery = velocity* * *πr*² is applied to Equation 2, a blood flow velocity of the corresponding blood vessel may be obtained by using the SIG as show in Equation 3 presented below:
[Equation 3] $Qartery = velocity ∗ {πr}^{2} ≒ {SIGπr}^{3}$

If *πr*² on both sides is removed in Equation 3, following Equation 4 is obtained:

[Equation 4] $Velocity = SIG ∗ r \left(unit, SI\right)$

In Equation 4, SIG uses a SIG (a maximum, a minimum, an average) of the corresponding blood vessel, and a blood vessel velocity obtained through Equation 4 is referred to as a SIG blood flow velocity (SIG-V).

As a result of performing a phantom study, SIG average values obtained by performing TOF MRA with two tubes having a diameter of 2.1 cm while maintaining average flow of 12.5±2.3, 8.5±2.6L/min were 2.2±0.4, 0.9±0.3 SI/min, respectively. In addition, SIG-BFs were 8.0±1.45, 3.27±1.09 SI· *cm*², respectively, and the SIG-BF and a real flow had a correlation coefficient of r=0.95, p<0.01. A radius of an inner diameter of a blood vessel is less than 1 cm, and an average radius of the aorta which is the largest artery of the human body is about 0.89 cm.

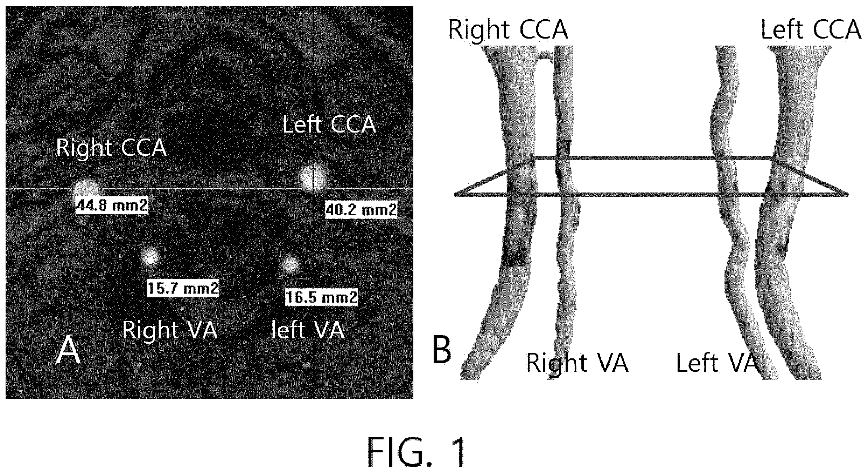

A correlation between blood flow of each blood vessel obtained through an ultrasound examination for the carotid arteries and the vertebral arteries in a clinical study, and a SIG-BF and a SIG-V obtained through TOF MRA was observed. An ultrasound examination was performed for 31 blood vessels of 8 healthy adults (a right vertebral artery of one person was not found). A time averaged mean flow velocity and a diameter were obtained by the ultrasound examination, and average blood flows of left and right common carotid arteries and left and right vertebral arteries obtained by the time averaged mean flow velocity and the diameter were 5.42±0.61, 6.34±1.34, 1.42±0.54, 0.88±0.37m³/sec, respectively. In addition, carotid TOF MRA was performed and SIG-BFs were acquired from four blood vessels according to Equation 2. Average SIG-BFs of left and right common carotid arteries and left and right vertebral arteries were 5.77±1.67, 5.95±0.48, 1.96±1.00, 0.89±0.45 SI·cm², respectively. Correlation coefficients between the two examinations were r=0.69 in the case of the left carotid artery, r=0.71 in the case of the right carotid artery, r=0.73 in the case of the left vertebral artery, and r=0.61 in the case of the right vertebral artery, all P values<0.01, and an inter-arterial correlation coefficient for all four blood vessels was 0.93 (P<0.001) (FIG. 1).

In addition, average blood flow velocities of the left and right common carotid arteries and the left and right vertebral arteries were 39.5±16.2, 41.1±11.8, 29.6±8.4, 27.1±10.7 cm/sec, respectively, and SIG-Vs were 16.34±4.0, 17.6±2.9, 13.1±3.5, 10.0±2.5 SI, respectively. Correlation coefficients between the two examinations of the blood vessels were r=0.47 in the case of the left carotid artery, r=0.68 in the case of the right carotid artery, r=0.63 in the case of the left vertebral artery, and r=0.64 in the case of the right vertebral artery, all P values<0.01, and an inter-arterial correlation coefficient for all four blood vessels was 0.67 (P<0.001).

### 2. SIG-tCBF

Since a cerebrovascular disease is indirectly or directly related to blood flow, quantitative analysis may be performed by obtaining a total cerebral blood flow (tCBF). An embodiment of the present disclosure proposes a method of obtaining a SIG-total cerebral blood flow (SIG-tCBF) by using a SIG-BF.

The SIG-tCBF is a value that is obtained by adding up SIG-BFs of four blood vessels, that is, two common carotid arteries (cca) and two vertebral arteries (va), and is defined as shown in Equation 5 presented below: [Equation 5] 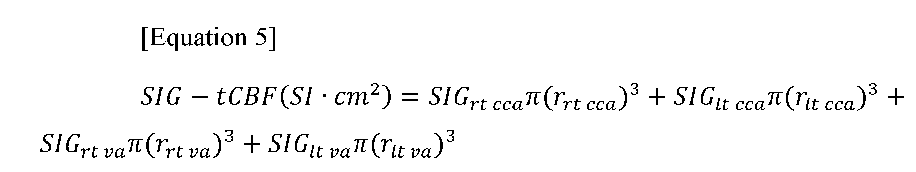 SIG - tCBF(SI · cm2) = SIGrt ccaπ(rrt cca)3 + SIGlt ccaπ(rlt cca)3 + SIGrt vaπ(rrt va)3 + SIGlt vaπ(rlt va)3
where *SIG_{rt cca}* is an SIG in the right common carotid artery, *r_{rt cca}* is a radius of the right common carotid artery, *SIG_{lt cca}* is an SIG in the left common carotid artery, *r_{lt cca}* is a radius of the left common carotid artery, *SIG_{rt va}* is an SIG in the right vertebral artery, *r_{rt va}* is a radius of the right vertebral artery, *SIG_{lt va}* is an SIG in the left vertebral artery, *r_{lt va}* is a radius of the left vertebral artery.

The clinical study showed that the SIG-tCBF has a high correlation with a total cerebral blood flow obtained through an ultrasound examination for the common carotid arteries and the vertebral arteries. The result of the ultrasound examination performed for 8 healthy adults showed that an average value of the total cerebral blood flow obtained by using the time averaged mean flow velocity was 13.5±1.4 cm³/sec. A SIG-total cerebral blood flow average value obtained after carotid TOF MRA was performed was 14.6±2.8 SI·cm². As a result of analyzing a correlation between the total cerebral blood flow obtained by the ultrasound examination, and the SIG-tCBF, a correlation coefficient was r=0.66, p<0.01 (FIG. 1).

FIG. 1 illustrates a SIG-tCBF measurement method utilizing MRA. View A of FIG. 1 is an axial (xy axis) cross-section view of two common carotid arteries and two vertebral arteries. From one cross section, cross-sectional areas of the four blood vessels may be obtained. View B of FIG. 1 shows SIG distribution of the common carotid arteries and the vertebral arteries, and a cross section indicated by lines is illustrated in view A of FIG. 1 (2D image). SIGs (SI/mm, meantSD) of the left and right common carotid arteries and the left and right vertebral arteries were 4.16±1.11, 4.01±0.70, 5.33±1.24, 5.56±0.91, respectively, and SIG-BFs (SI·cm²) were 3.63, 4.01, 0.88, 0.92, respectively. A SIG-tCBF was 9.45 SI·cm². For reference, the blood flows of the left and right common carotid arteries and the left and right vertebral arteries obtained by carotid ultrasound were 4.33, 4.67, 0.85, 0.99 cm³/sec, respectively, and the total cerebral blood flow was 10.85 cm³/sec.

### 3. SIG-pCBF/SIG-colBF

As distinct from the carotid TOF MRA, intracranial TOF MRA is angiography that is most widely used in a clinical setting. However, the intracranial TOF MRA does not scan proximal portions of the common carotid artery or vertebral artery, but images the internal carotid artery and blood vessels of distal portions thereof (the middle cerebral artery, the anterior cerebral artery), and distal portions of the vertebral artery (V4 portions) and the basilar artery. Like the carotid TOF MRA, the intracranial TOF MRA may be inappropriate for inducing the total cerebral blood flow, but may acquire only blood flow of blood flowing toward the cerebral ventricle and the brain stem through the anterior, middle, posterior cerebral arteries and the basilar artery.

This method selectively acquires only blood flow of blood flowing toward the brain stem and the cerebral ventricle, except for blood flow of blood toward the external carotid artery and the ophthalmic artery, by utilizing of SIG-BFs of four places, specifically, the internal carotid arteries before the final branch of the internal carotid artery, that is, before the branch of the anterior cerebral artery and the middle cerebral artery, and the left and right vertebral arteries before the branch of the posterior inferior cerebellar artery. The blood flow obtained in this way is referred to as a SIG-parenchymal cerebral blood flow (SIG-pCBF), which is defined as shown in Equation 6 presented below: [Equation 6] 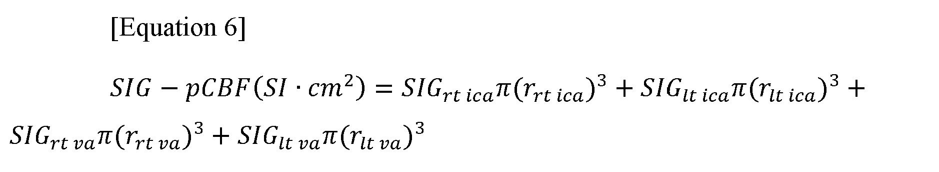 SIG - pCBF(SI · cm2) = SIGrt icaπ(rrt ica)3 + SIGlt icaπ(rlt ica)3 + SIGrt vaπ(rrt va)3 + SIGlt vaπ(rlt va)3
where *SIG_{rt ica}* and *r_{rt ica}* are an SIG and a radius at a terminal end of the right internal carotid artery before the branch of the anterior cerebral artery and the middle cerebral artery, *SIG_{lt ica}* and *r_{lt ica}* are an SIG and a radius at a terminal end of the left internal carotid artery before the branch of the anterior cerebral artery and the middle cerebral artery, *SIG_{rt va}* and *r_{rt va}* are an SIG and a radius at the right vertebral artery before the branch of the posterior inferior cerebellar artery, and *SIG_{lt va}* and *r_{lt va}* are an SIG and a radius at the left vertebral artery before the branch of the posterior inferior cerebellar artery.

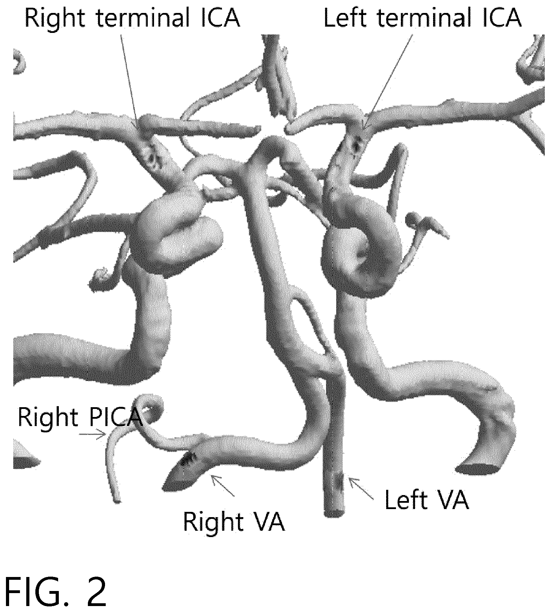

FIG. 2 illustrates an example showing SIG-pCBF. The SIG-pCBF is acquired by adding up SIG-BFs at four places, the terminal ends of the left and right internal carotid arteries and the left and right vertebral arteries (anterior portions of the origin of the posterior inferior cerebellar artery). FIG. 2 shows a result of performing TOF MRA for healthy adults. The result shows that average SIGs of the left and right internal carotid arteries and the left and right vertebral arteries were 6.86, 8.48, 9.67, 11.2 SI/mm, respectively, and SIG-BFs (SI·cm²) were 1.24, 1.25, 1.30, 0.73, respectively, indicating that the cerebrum had the same blood flow on the left and the right, and the cerebellum and the brain stem had a larger amount of blood flow on the right vertebral artery. The SIG-pCBF was 4.52 SI·cm².

A SIG-collateral blood flow (SIG-colBF) of blood going toward the ophthalmic artery and the external carotid artery may be obtained by deducting the SIG-pCBF from the above-described SIG-tCBF as follows:

[Equation 7] $SIG - colBF \left(SI⋅{cm}^{2}\right) = SIG - tCBF - SIG - pCBF$

### 4. Δ SIG ratio/SIG-BF ratio

Cerebral hemorrhage is mostly divided into three types: deep intracerebral hemorrhage, subarachnoid hemorrhage, and traumatic cerebral hemorrhage. It is known that the deep intracerebral hemorrhage has characteristics highly related with the lacunar infarction or a small vessel disease, and the subarachnoid hemorrhage is caused by rupture of an aneurysm.

The subarachnoid hemorrhage has a symptom of a beastly headache and a loss of consciousness, and is a serious disease that requires immediate surgery or intensive care. The aneurysm is a potential risk factor that may cause subarachnoid hemorrhage, and, when the aneurysm is found, a very considerate clinical decision may be required even when subarachnoid hemorrhage does not occur. That is, it should be determined whether the aneurysm is treated through surgery or angioplasty using a stent, or is monitored through a clinical course.

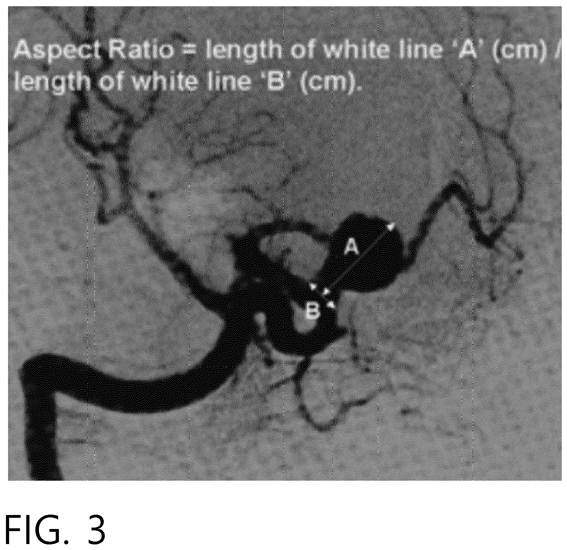

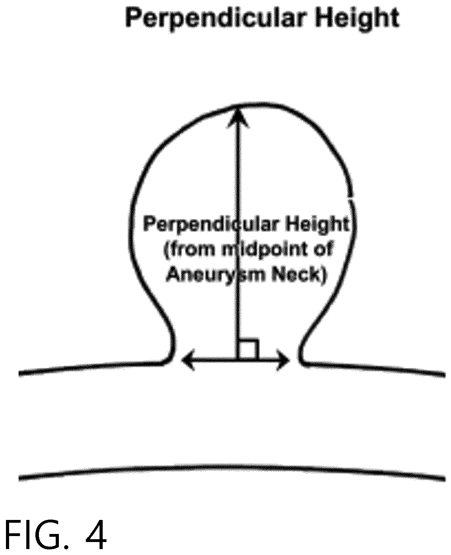

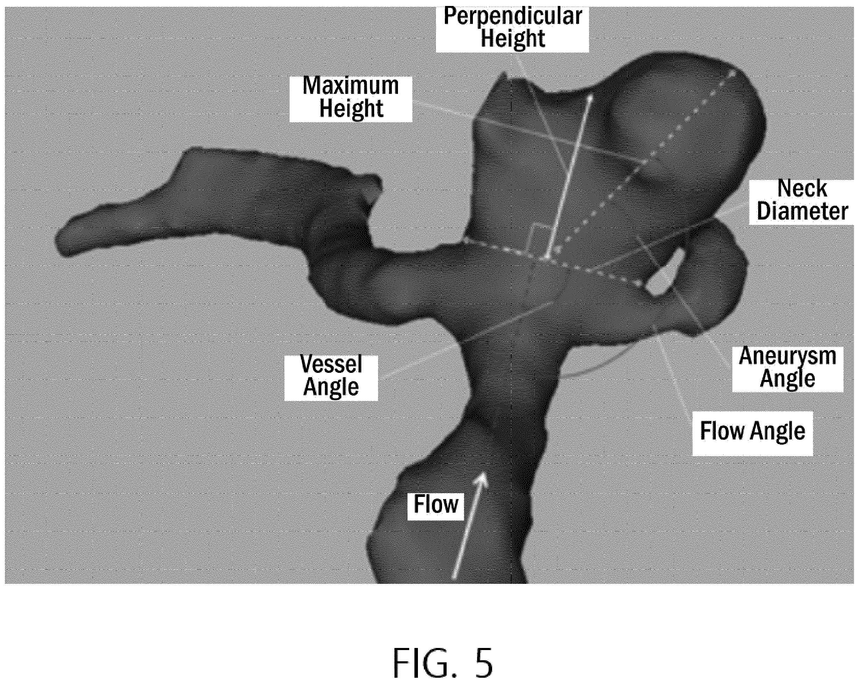

A related-art aneurysm classification method is mainly based on measurement of an appearance or shape. Through many analyses, it has been reported that a size, a shape, an aspect ratio (FIGS. 3, 4), the presence of bleb, a flow angle (FIG. 5) are major risk factors of rupture of an aneurysm. In a study using a computer simulation (computation fluid dynamics (CFD)), it has been reported that a cerebral aneurysm having a low shear stress has a high risk of rupture. However, it is difficult to accurately measure a 3D aneurysm as a 2D concept having a length or an incidence angle, and there is a high risk of different opinions between measurers. In addition, a computer simulation such as CFD may have difficulty in acquiring reliable results by giving the same environment/technique to all patients while satisfying various conditions, such as inlet/outlet adjustment suitable for each patient and appropriate 3D surface setting, etc.

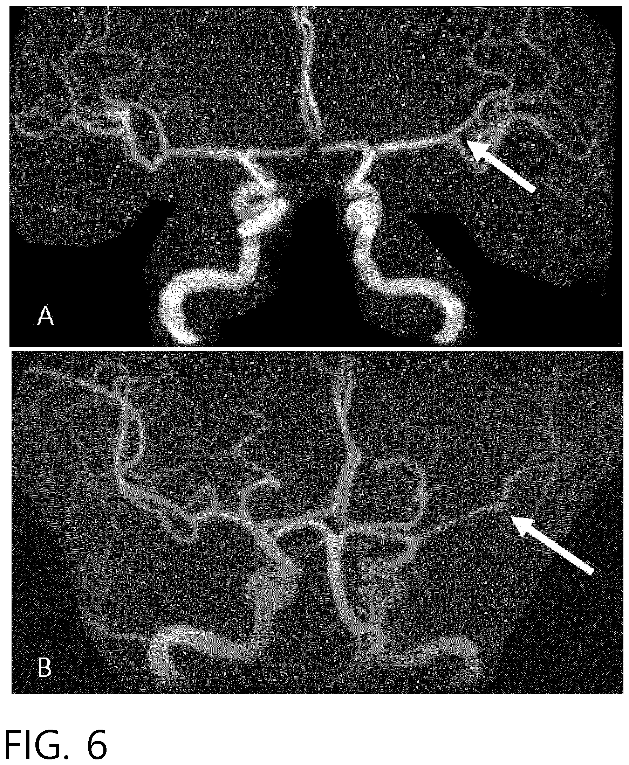

The principal result of all angiography techniques including existing TOF MRA is diagnosis for determining the existence of an aneurysm. Precedent researches on hemodynamic methods for indicating the risk of rupture of an aneurysm have been reported, but there is hardly any method for using for screening in a real clinical setting. For example, view (A) of FIG. 6 illustrates a result of imaging from a 74-year-old asymptomatic woman, and view (B) of FIG. 6 illustrates a result of imaging from a 37-year-old man having subarachnoid hemorrhage. In both cases, an aneurysm is observed from an M2 branch of the left middle cerebral artery, but it is difficult to predict the risk of rupture only with a vascular image.

However, the method using an MRA SIG and a SIG-BF, which is proposed in embodiments of the present disclosure, may overcome various problems of related-art methods, and may obtain reliable results from all patients and may be utilized for predicting risks.

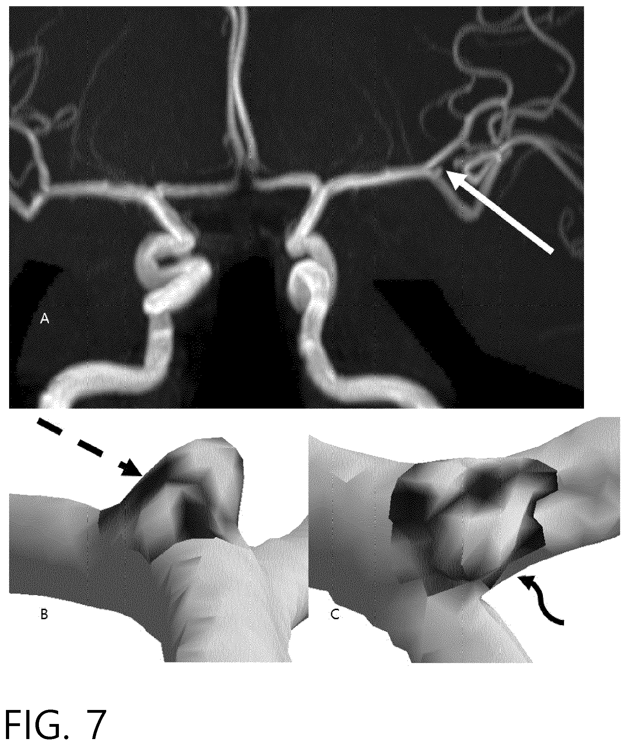

As a result of analyzing MRA SIGs of unruptured cerebral aneurysms of 80 persons, each aneurysm may be divided into a part that receives inflowing blood, and the other part that does not receive inflowing blood. That is, it may be identified that an aneurysm is divided into a blood inflow portion and a recirculation portion of inflowing blood. FIG. 7 illustrates a result of performing TOF MRA for a 56-year-old woman patient having an asymptomatic aneurysm. As shown in view A of FIG. 7, an aneurysm is observed from an M2 origin portion of the left middle cerebral artery as shown in view A of FIG. 7 (indicated by the arrow). Views B and C of FIG. 7 illustrate results of SIG analysis regarding an aneurysm of the left middle cerebral artery of view A. One side of the aneurysm may be divided into a portion that shows a high SIG value (blood inflow portion, the broken arrow) and the opposite portion that shows a low value (blood recirculation portion, the curved arrow). View C of FIG. 7 illustrates an image when viewed from above.

In determining the risk of rupture of an aneurysm, it is important to find out whether the aneurysm is or will be subjected to strong pressure. Based on characteristics described in FIG. 7, it may be determined that the risk of rupture of an aneurysm is high when the SIG of the blood inflow portion is high (blood is easy to flow) and there is an area (rupture risk portion) having a very low value in the blood recirculation portion, and in this case, it may be regarded that an amount of blood in the aneurysm is large.

For quantitative analysis of the risk of rupture of a cerebral aneurysm, the present disclosure provides concepts of Δ SIG ratio (a ratio of a maximum difference value of SIG) and a SIG-BF ratio.

Δ SIG ratio is defined by Equation 8 presented below:

[Equation 8] $Δ SIG ratio = \left(Δ SIG-aneurysm\right) / \left(Δ SIG-preaneurysm\right)$
where Δ SIG-aneurysm is a difference between a maximum SIG and a minimum SIG of an aneurysm, and Δ SIG-preaneurysm is a difference between a maximum SIG and a minimum SIG of a normal blood vessel of a proximal portion of an aneurysm.

The SIG-BF ratio is defined by Equation 9 presented below:

[Equation 9] $SIG - BF ratio = \left(SIG-BF-aneurysm\right) / \left(SIG-BF-preaneurysm\right)$
where SIG-BF-aneurysm is a SIG-BF at a point showing a maximum radius in an aneurysm, and SIG-BF-preaneurysm is a SIG-BF of a normal blood vessel of a proximal portion of an aneurysm.

As a result of analyzing SIGs and SIG-BFs of all normal portions of an artery and the aneurysm, an average Δ SIG ratio of an asymptomatic group was 0.58, and a SIG-BF ratio thereof was 2.47. On the other hand, an average Δ SIG ratio of two patients having rupture was 1.39 and a SIG-BF ratio thereof was 11.64, which greatly differ from the asymptomatic group. In addition, an area corresponding to a low SIG corresponding to a lower rank 20% tile in the aneurysm was 2.81 mm² in the asymptomatic group and was 9.43 mm² in the rupture group. In the case of the rupture group, a blood recirculation portion having a low SIG is larger.

### 5. Cerebrovascular disease risk analysis system

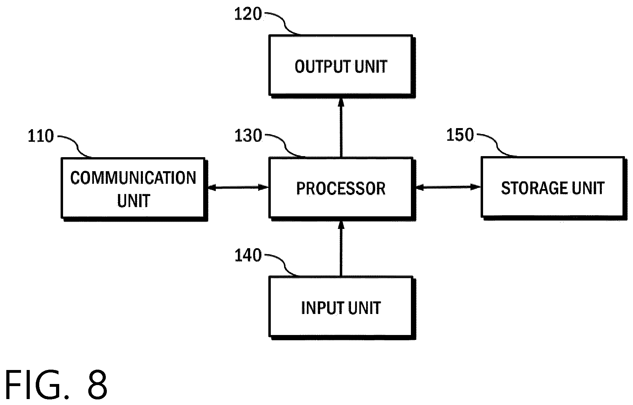

FIG. 8 is a block diagram of a cerebrovascular disease risk analysis system according to an embodiment of the present disclosure. The cerebrovascular disease risk analysis system according to an embodiment of the present disclosure is a system for predicting a risk of a cerebrovascular disease by calculating a SIG-BF and factors related thereto by using a SIG of a blood vessel site in an MRA image.

The cerebrovascular disease risk analysis system according to an embodiment of the disclosure may be implemented by a computing system which includes a communication unit 110, an output unit 120, a processor 130, an input unit 140, and a storage unit 150 as shown in the drawing.

The communication unit 110 is a means for communicating with an external device and connecting to an external network, and receives an MRA image of a patient from an MRA device in an embodiment of the disclosure.

The processor 130 may calculate a SIG-BF, a SIG-V, a SIG-tCBF, a SIG-pCBF, a SIG-colBF, a Δ SIG ratio and a SIG-BF ratio of a blood vessel site from the MRA image received through the communication unit 110.

In addition, the processor 130 may predict a risk of a cerebrovascular disease by using the calculated factors. Specifically, the processor 130 may predict the risk of the cerebrovascular disease based on a correlation between a factor and the cerebrovascular disease.

There is no limit to types of factors and the number of factors used for predicting the risk of the cerebrovascular disease.

The output unit 120 is a display that displays factor values calculated by the processor 130 and the predicted risk of the cerebrovascular disease, and the input unit 140 is a means for receiving a user command.

The storage unit 150 provides a storage space that is necessary for the processor 130 to calculate factors and predict the risk of the cerebrovascular disease.

### 6. Variation

Up to now, a method and a system for calculating a SIG-BF of a blood vessel site by using a SIG in an MRA image, and predicting a risk of a cerebrovascular disease from calculated factors have been described with reference to preferred embodiments.

In the above-described embodiments, the factors derived from the SIG of the blood vessel in the MRA image may be a SIG-BF, a SIG-V, a SIG-tCBF, a SIG-pCBF, a SIG-colBF, a Δ SIG ratio and a SIG-BF ratio.

The technical concept of the present disclosure may be applied to a computer-readable recording medium which records a computer program for performing the functions of the apparatus and the method according to the present embodiments. In addition, the technical idea according to various embodiments of the present disclosure may be implemented in the form of a computer readable code recorded on the computer-readable recording medium. The computer-readable recording medium may be any data storage device that can be read by a computer and can store data. For example, the computer-readable recording medium may be a read only memory (ROM), a random access memory (RAM), a CD-ROM, a magnetic tape, a floppy disk, an optical disk, a hard disk drive, or the like. A computer readable code or program that is stored in the computer readable recording medium may be transmitted via a network connected between computers.

In addition, while preferred embodiments of the present disclosure have been illustrated and described, the present disclosure is not limited to the above-described specific embodiments. Various changes can be made by a person skilled in the art without departing from the scope of the present invention which is defined in the claims.

## Claims

1. A system for analyzing a risk of a rupture of a cerebral aneurysm, the system comprising:
a processor (130) configured to calculate a plurality of factors from an MRA image, and configured to predict the risk of the rupture of the cerebral aneurysm by using the calculated factors;
an output unit (120) configured to output a result of calculating by the processor and a result of predicting; and
a storage unit (150) configured to provide a storage space necessary for the processor;
**characterised in that** the plurality of factors includes:
a ΔSIG ratio of the blood vessel site in the MRA image calculated using the following equation: ΔSIG ratio=(ΔSIG-aneurysm)/(ΔSIG-preaneurysm), where ΔSIG-aneurysm is a difference between a maximum signal intensity gradient and a minimum signal intensity gradient of an aneurysm, and ΔSIG-preaneurysm is a difference between a maximum signal intensity gradient and a minimum signal intensity gradient of a normal blood vessel of a proximal portion of the aneurysm; and
a SIG-BF ratio calculated using the following equation: SIG-BF ratio=(SIG-BF-aneurysm)/(SIG-BF-preaneurysm), where SIG-BF-aneurysm is a SIG-BF of the aneurysm showing a maximum radius, and SIG-BF-preaneurysm is a SIG-BF of a normal blood vessel of a proximal portion of the aneurysm;
wherein SIG-BF is a signal intensity gradient-blood flow of a blood vessel site in the MRA image calculated using the following equation: SIG-BF= *SIGπr*³ where SIG is a signal intensity gradient in the blood vessel site and r is a radius of the blood vessel.

2. The system of claim 1, wherein the plurality of factors further includes a SIG-velocity (SIG-V) of the blood vessel site in the MRA image calculated using the following equation: $SIG - Velocity = SIG * r$ where SIG is the signal intensity gradient in the blood vessel site and r is the radius of the blood vessel.

3. The system of claim 1 or 2, wherein the SIG is any one of a maximum value, a minimum value, and an average value of the signal intensity gradient.

## Patentansprüche

1. System zum Analysieren eines Risikos einer Ruptur eines zerebralen Aneurysmas, wobei das System Folgendes umfasst:
einen Prozessor (130), der dazu konfiguriert ist, eine Vielzahl von Faktoren aus einem MRA-Bild zu berechnen, und dazu konfiguriert ist, das Risiko der Ruptur des zerebralen Aneurysmas unter Verwendung der berechneten Faktoren vorherzusagen;
eine Ausgabeeinheit (120), die dazu konfiguriert ist, ein Ergebnis des Berechnens durch den Prozessor und ein Ergebnis des Vorhersagens auszugeben; und
eine Speichereinheit (150), die dazu konfiguriert ist, einen Speicherplatz bereitzustellen, der für den Prozessor erforderlich ist;
**dadurch gekennzeichnet, dass** die Vielzahl von Faktoren Folgendes enthält:
ein ΔSIG-Verhältnis der Blutgefäßstelle in dem MRA-Bild, das unter Verwendung der folgenden Gleichung berechnet wird: ΔSIG-Verhältnis =(ΔSIG-Aneurysma)/(ΔSIG-Präaneurysma), wobei ΔSIG-Aneurysma eine Differenz zwischen einem maximalen Signalintensitätsgradienten und einem minimalen Signalintensitätsgradienten eines Aneurysmas ist und ΔSIG-Präaneurysma eine Differenz zwischen einem maximalen Signalintensitätsgradienten und einem minimalen Signalintensitätsgradienten eines normalen Blutgefäßes eines proximalen Abschnitts des Aneurysmas ist; und
ein SIG-BF-Verhältnis, das unter Verwendung der folgenden Gleichung berechnet wird: SIG-BF-Verhältnis =(SIG-BF-Aneurysma)/(SIG-BF-Präaneurysma), wobei SIG-BF-Aneurysma ein SIG-BF des Aneurysmas ist, das einen maximalen Radius zeigt, und SIG-BF-Präaneurysma ein SIG-BF eines normalen Blutgefäßes eines proximalen Abschnittes des Aneurysmas ist;
wobei SIG-BF ein Signalintensitätsgradienten-Blutfluss einer Blutgefäßstelle in dem MRA-Bild ist, der unter Verwendung der folgenden Gleichung berechnet wird: SIG-BF=*SIGπr³*, wobei SIG ein Signalintensitätsgradient an der Blutgefäßstelle ist und r ein Radius des Blutgefäßes ist.

2. System nach Anspruch 1, wobei die Vielzahl von Faktoren ferner eine SIG-Geschwindigkeit (SIG-V) der Blutgefäßstelle in dem MRA-Bild enthält, die unter Verwendung der folgenden Gleichung berechnet wird: $SIG - Geschwindigkeit = SIG * r$ wobei SIG der Signalintensitätsgradient an der Blutgefäßstelle ist und *r* der Radius des Blutgefäßes ist.

3. System nach Anspruch 1 oder 2, wobei der SIG einer von einem Maximalwert, einem Minimalwert und einem Durchschnittswert des Signalintensitätsgradienten ist.

## Revendications

1. Système permettant l'analyse d'un risque de rupture d'un anévrisme cérébral, le système comprenant :
un processeur (130) configuré pour calculer une pluralité de facteurs à partir d'une image d'ARM, et configuré pour prédire le risque de rupture de l'anévrisme cérébral à l'aide des facteurs calculés ;
une unité de sortie (120) configurée pour produire un résultat de calcul au moyen du processeur et un résultat de prédiction ; et
une unité de stockage (150) configurée pour fournir un espace de stockage nécessaire au processeur ;
**caractérisé en ce que** la pluralité de facteurs comprend :
un rapport ΔSIG du site de vaisseau sanguin dans l'image d'ARM calculée à l'aide de l'équation suivante : rapport ΔSIG = (ΔSIG-anévrisme)/(ΔSIG-préanévrisme), où ΔSIG-anévrisme représente une différence entre un gradient d'intensité de signal maximal et un gradient d'intensité de signal minimal d'un anévrisme, et ΔSIG-préanévrisme représente une différence entre un gradient d'intensité de signal maximal et un gradient d'intensité de signal minimal d'un vaisseau sanguin normal d'une partie proximale de l'anévrisme ; et
un rapport SIG-BF calculé à l'aide de l'équation suivante : rapport SIG-BF = (SIG-BF-anévrisme)/(SIG-BF-préanévrisme), où SIG-BF-anévrisme représente un SIG-BF de l'anévrisme montrant un rayon maximal, et SIG-BF-préanévrisme représente un SIG-BF d'un vaisseau sanguin normal d'une partie proximale de l'anévrisme ;
dans lequel SIG-BF représente un gradient d'intensité de signal-flux sanguin d'un site de vaisseau sanguin dans l'image d'ARM calculée à l'aide de l'équation suivante : SIG-BF = *SIGπr³* où SIG représente un gradient d'intensité de signal dans le site de vaisseau sanguin et r représente un rayon du vaisseau sanguin.

2. Système selon la revendication 1, ladite pluralité de facteurs comprenant en outre une SIG-vitesse (SIG-V) du site de vaisseau sanguin dans l'image d'ARM calculée à l'aide de l'équation suivante : $SIG - Vitesse = SIG * r$ où SIG représente le gradient d'intensité de signal dans le site du vaisseau sanguin et *r* représente le rayon du vaisseau sanguin.

3. Système selon la revendication 1 ou 2, ledit SIG étant une valeur quelconque parmi une valeur maximale, une valeur minimale et une valeur moyenne du gradient d'intensité de signal.
